# EUROPEAN PATENT SPECIFICATION

(11) **EP 1 703 845 B1**
(45) Date of publication and mention of the grant of the patent: **23.01.2008**
(21) Application number: 04815449.6
(22) Date of filing: 21.12.2004
(51) Int. Cl.: A61B 17/00

(54) **APPARATUS FOR DELIVERING HAEMOSTATIC SEALING MATERIALS**
GERÄT ZUR ABGABE VON HÄMOSTATISCHEN DICHTUNGSMITTELN
DISPOSITIF POUR LA DÉLIVRANCE DES AGENTS HÉMOSTATIQUES

(30) Priority: 24.12.2003 US 745946; 27.10.2004 US 975205
(43) Date of publication of application: 27.09.2006
(73) Proprietor: Accessclosure, Inc., Palo Alto, CA 94303 (US)
(72) Inventor: KHOSRAVI, Farhad, Los Altos Hills, CA 94022 (US); BAGAOISAN, Celso, J., Union City, CA 94587 (US); SURESH, Pai, S., Mountain View, CA 94043 (US); SERSHEN, Scott, R., Belmont, CA 94002 (US)
(74) Representative: Weydert, Robert
(86) International application number: PCT/US2004/043374
(87) International publication number: WO 2005/063129

(56) References cited:
- EP-A- 0 716 833
- WO-A-00/06029
- WO-A-94/28798
- WO-A-03/094749
- US-A- 5 728 132
- US-B1- 6 302 898

## Description

### FIELD OF INVENTION

This invention relates generally to apparatus for sealing punctures in a body, and, more particularly, to apparatus for facilitating hemostasis of a vascular puncture extending through tissue into a blood vessel.

### BACKGROUND

Apparatus are known for accessing a patient's vasculature percutaneously for performing a procedure within the vasculature, and for sealing the puncture that results after completing the procedure. For example, a hollow needle may be inserted through a patient's skin and overlying tissue into a blood vessel. A guidewire is then passed through the needle into the blood vessel, whereupon the needle is removed. An introducer sheath is then advanced over the guidewire into the vessel, e.g., in conjunction with or subsequent to one or more dilators. A catheter or other device may be advanced through the introducer sheath and over the guidewire into a position for performing a medical procedure within the patient's body. In this manner, the introducer sheath facilitates introducing various instruments into the vessel, while minimizing trauma to the vessel wall and blood loss.

Upon completing the procedure, the instrument(s) and introducer sheath are removed, leaving a puncture extending between the skin and the vessel. To seal the puncture, external pressure may be applied to the overlying tissue, e.g., manually and/or using sandbags, until hemostasis occurs. This procedure, however, can be time consuming and expensive, requiring as much as an hour of a medical professional's time. It is also uncomfortable for the patient, and may require the patient to remain immobilized in an operating room, catheter lab, or holding area. In addition, a risk of hematoma exists from bleeding before hemostasis occurs.

Various apparatus have been suggested for sealing a percutaneous puncture instead of or in addition to using external pressure. For example, U.S. Patent No. 5,108,421 to Fowler discloses using a collagen plug that is delivered into a puncture through tissue. After completing the procedure, the introducer sheath and/or guidewire used to access the patient's vasculature via the puncture are removed. In one embodiment, a catheter is inserted through the puncture into the blood vessel. A balloon on the catheter is expanded and then retracted until the balloon is disposed adjacent the puncture at the wall of the vessel. A plug is then advanced into the puncture until the plug contacts the balloon, thereby preventing the plug from entering the vessel. Once the plug is positioned within the puncture, the balloon is deflated and withdrawn, leaving the plug to expand and seal the puncture and/or promote hemostasis.

WO 03/094749 A1 discloses a system for sealing a puncture extending through tissue to a body lumen of a patient according to the preamble of claim 1.

### SUMMARY OF THE INVENTION

This invention is directed to a system according to claim 1. Preferably, the expandable member is biased towards the enlarged condition, and the system includes a removable constraint for maintaining the expandable member in the contracted condition. For example, the constraint may include a tubular member slidable over the elongate member, e.g., to cover the expandable member (e.g., to maintain the expandable member in the contracted condition), and uncover the expandable member (e.g., to allow the expandable member to expand towards the enlarged condition). Alternatively, the expandable member may be selectively expandable, e.g., by activating an actuator on a proximal end of the elongate member. In exemplary embodiments, the expandable may be a mesh structure, an expandable frame, and the like, e.g., including a substantially nonporous covering on at least a portion thereof.

### BRIEF DESCRIPTION OF THE DRAWINGS

The drawings illustrate exemplary,embodiments of the invention, in which:
FIGS. 1A-1C are side views of components of an embodiment of an apparatus for sealing a puncture extending through tissue, including a delivery sheath, a retaining sheath, and a guidewire with self-expanding tamp, respectively.
FIGS. 2A and 2B are side views of a retaining sheath received over a guidewire with an expandable tamp, with the retaining sheath retracted and extended such that the tamp is in contracted and enlarged conditions, respectively.
FIGS. 3A-3E are cross-sectional views of a patient's body, illustrating a method using apparatus of the invention for sealing a puncture extending between the patient's skin and a blood vessel, using the system of FIGS. 1A-1C.
FIGS. 4A and 4B are side views of another embodiment of a guidewire with an expandable tamp in contracted and enlarged conditions, respectively.

### DETAILED DESCRIPTION OF THE ILLUSTRATED EMBODIMENTS

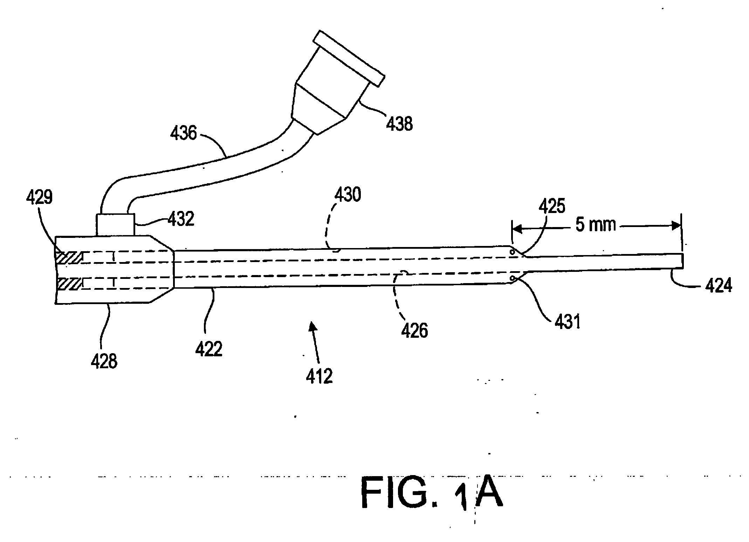

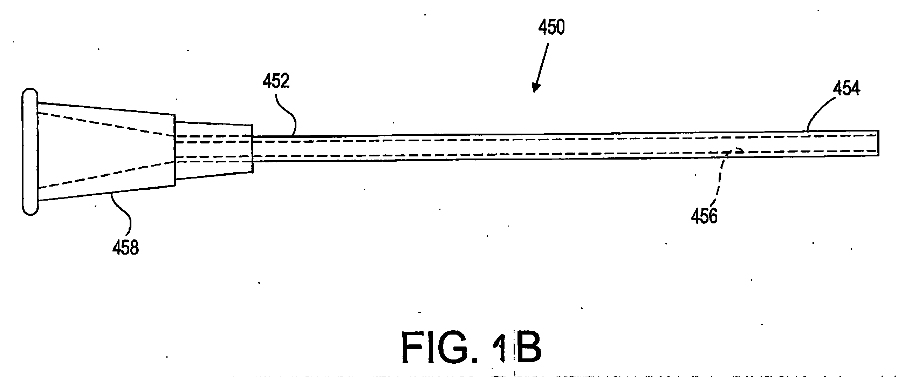

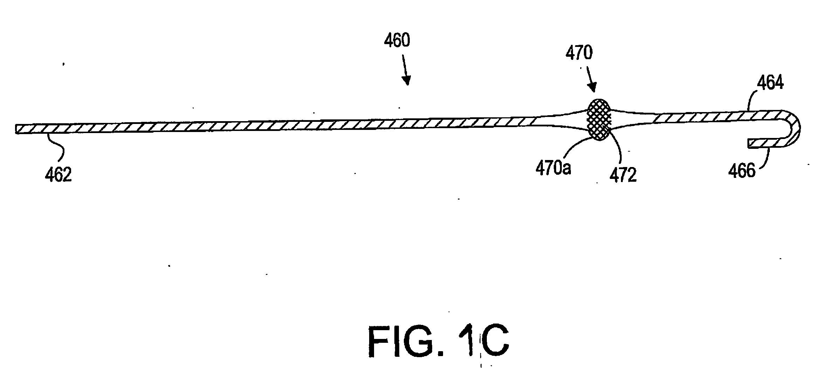

Turning to FIGS. 1A-1C, an embodiment of a system 410 is shown for sealing a puncture through tissue that generally includes a delivery or injection sheath 412, a retaining sheath 450, a source of sealing compound, e.g., a dual syringe assembly (not shown), and a guidewire 460 having an expandable tamp 470 thereon. Optionally, the system 410 may include a needle, and/or an introducer sheath.

The delivery sheath 412 includes a proximal end 422, a distal end 424, a primary or guidewire lumen 426 extending between the proximal and distal ends 422, 424, and, one or more secondary lumens 430. The delivery sheath 412 includes one or more outlets 431 in the wall of the delivery sheath 412, e.g., at a tapered intermediate portion 425 between the proximal and distal ends 422, 424. In an exemplary embodiment, two outlets 431 are located about five millimeters (5 mm) from the distal end 424.

A housing 428 may be attached to or otherwise provided on the proximal end 422 of the delivery sheath 412, including a side port 432 (or optionally multiple side ports, not shown) that communicates with an interior of the housing 428 and the secondary lumen 430. The housing 428 may include one or more seals 429 to seal the interior of the housing 428 such that sealing compound delivered via the side port 432 may be directed through the secondary lumen 430. The housing 428 may also include one or more seals (not shown), e.g., a hemostatic seal, for sealing the primary lumen 426. Tubing 436 may be connected to or otherwise extend from the side port 432 to a luer lock adapter 438.

Turning to FIG. 1B, the retaining sheath 450 may be an elongate tubular member including a proximal end 452, a distal end 454, and a lumen 456 extending therebetween. A hub 458 may be located on the proximal end 452, e.g., to facilitate manipulating the retaining sheath 450. The retaining sheath 450 may have a diameter or other size to allow the distal end 454 to be inserted into and/or through the lumen 426 of the delivery sheath 412, while the hub 458 may be larger than the size of the lumen 426, e.g., to provide a stop limiting distal advancement of the retaining sheath 450 into the delivery sheath 412. The retaining sheath 450 may be sufficiently flexible to conform to the surrounding anatomy, e.g., when the retaining sheath 450 is inserted into or removed from a puncture, e.g., along with other components, such as the guidewire 460.

Turning to FIG. 1C, the guidewire 460 may be an elongate member including a proximal end 462 and a distal end 464, e.g., including a "J" tip 466. The guidewire 460 may be formed from a solid wire, one or more coiled wires, and/or from a solid-walled tube, similar to conventional guidewires. The guidewire 460 may be formed from a variety of known materials, e.g., metals, such as stainless steel or Nitinol, plastics, and the like. Thus, the guidewire 460 may be sufficiently flexible to navigate tortuous anatomy, but may have sufficient column strength to be pushable from the proximal end 462.

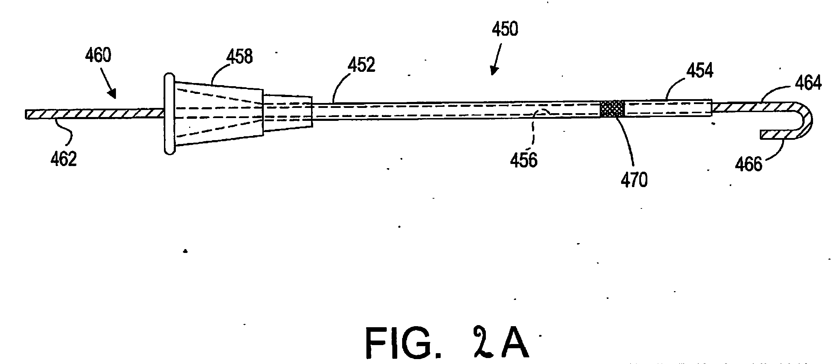

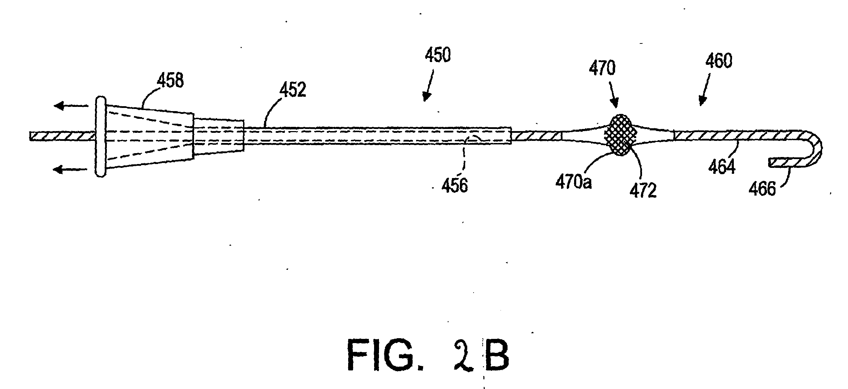

The tamp 470 may an expandable structure adjacent the distal tip 466 that may be biased towards an enlarged condition, as shown in FIGS. 1C and 2B, but may be resiliently compressible towards a contracted condition, as shown in FIG. 2A. In the embodiment shown in FIG. 1C, the tamp 470 includes a braided mesh of wires or other fibers 472 that assume a generally spherical or elliptical disk shape in the enlarged condition.

The fibers 472 may be formed from a shape memory material, e.g., Nitinol, stainless steel, a polymer or other plastic, and the like, that has the enlarged condition programmed into the fibers 472, e.g., by heat treatment. Thus, the fibers 472 may be elastically (or superelastically) deformed, e.g., compressed into the contracted condition using the retaining sheath 450, yet resiliently expand towards the enlarged condition once released, as explained further below The tamp 470 may shorten as it expands from the contracted condition towards the enlarged condition, and may lengthen again as it is compressed back towards the contracted condition.

The fibers 472 may include a coating, cover, or other skin (not shown) that covers all or a portion of the tamp 470. For example, at least the proximal portion 470a of the tamp 470 may include a coating or other skin that extends across the spaces between the fibers 472 such that the proximal portion 470a is substantially nonporous. Alternatively, all of the tamp 470 may include a coating or other skin.

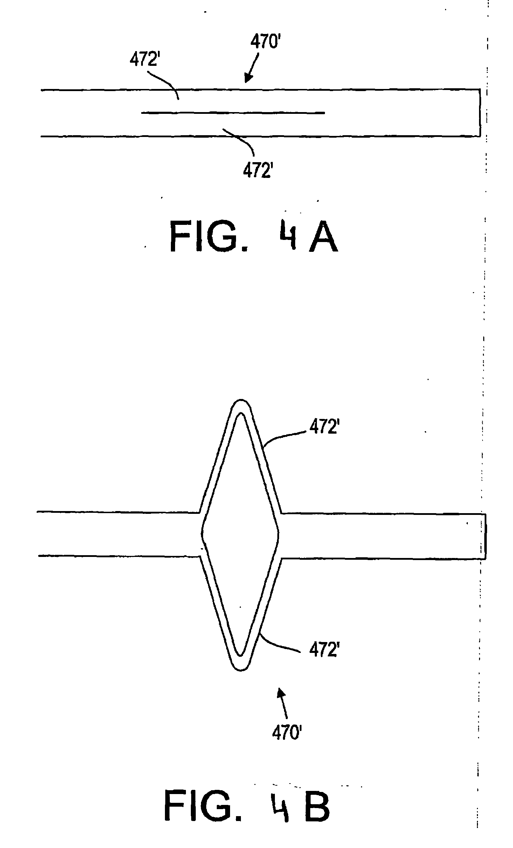

In a further alternative, as shown in FIGS. 4A and 4B, the tamp 470' may include a plurality of struts 472' that are expandable between enlarged and contracted conditions. The struts 472' may extend substantially axially in the contracted condition and may buckle at an intermediate location thereon as they expand radially outwardly towards the enlarged condition. In one embodiment, the struts 472' may be biased towards the enlarged condition, yet may be resiliently compressed towards the contracted condition, similar to the tamp 470 of FIG. 1C.

Alternatively, the struts 472' may be selectively expanded and/or compressed, e.g., using an internal pull wire or other actuator (not shown). Exemplary embodiments of expandable strut and/or mesh structures, e.g., which may include coatings or skins, are disclosed in U.S. Patent Nos. 6,238,412 and 6,635,068, and in application Publication No. US 2003/0078616.

Similar to the mesh tamp 470, the struts 472' may include a coating or other skin, e.g., to extend between struts to provide at least a substantially nonporous proximal portion. In addition, the axial dimension of the struts 472' may shorten as the struts 472' move from the contracted condition to the enlarged condition, similar to the mesh tamp 470.

Turning to FIGS. 2A and 2B, the guidewire 460 of FIG. 1C may be inserted into the lumen 456 of the retaining sheath 450 of FIG. 1B. Thus, the retaining sheath 450 may be slidable axially along the guidewire 460, e.g., to selectively cover and uncover the tamp 470. When the retaining sheath 450 is directed over the tamp 470, as shown in FIG. 2A, the tamp 470 may be compressed towards and/or maintained in the contracted condition. When the retaining sheath 450 is retracted to expose the tamp 470, as shown in FIG. 2B, the tamp 470 may automatically expand towards the enlarged condition.

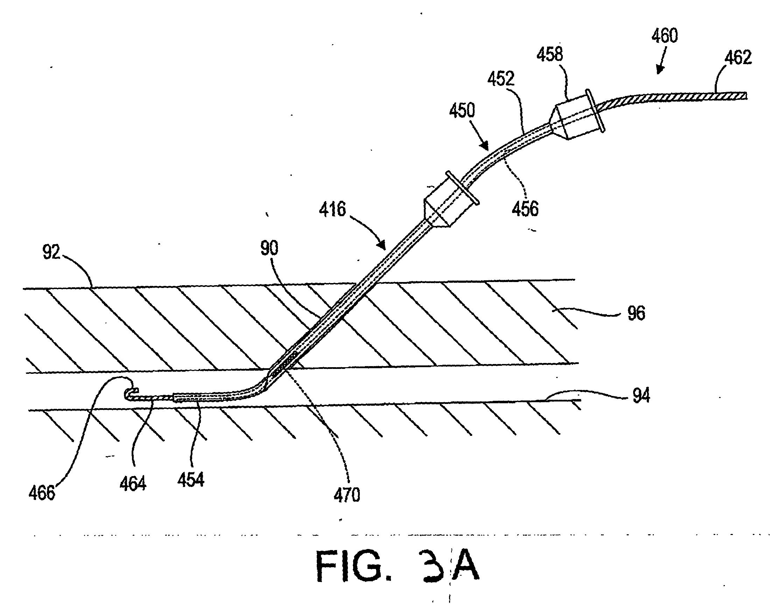

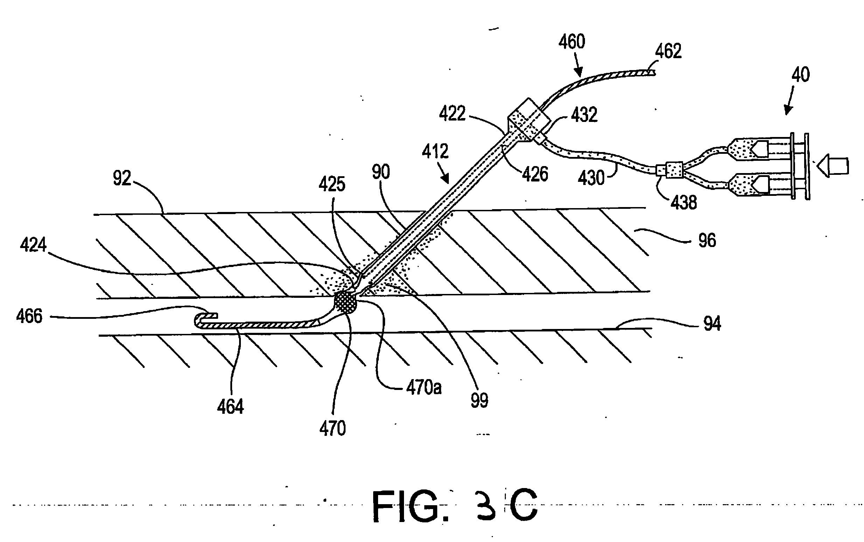

Turning to FIGS. 3A-3E, a method using apparatus of the invention is shown for sealing a puncture 90 extending from a patient's skin 92 to a body lumen, e.g., a blood vessel 94. Initially, as shown in FIG. 3A, a hollow needle 416 may be inserted through the patient's skin 92 to create the puncture 90 through intervening tissue 96, and into the vessel 94.

The guidewire 460 and retaining sheath 450 may be inserted into the puncture 90, e.g., through the needle 416. until the distal tip 466 is disposed within the vessel 94. As shown, the retaining sheath 450 covers the tamp 470 on the guidewire 460 as the guidewire 460 is advanced through the needle 416, thereby maintaining the tamp 470 in the contracted condition. Thus, the guidewire 460 may be advanced through the needle 416 until the tamp 470 is disposed within the vessel 94. Alternatively, it may be possible to compress the tamp 470 and insert it into the needle 416 without the retaining sheath 460.

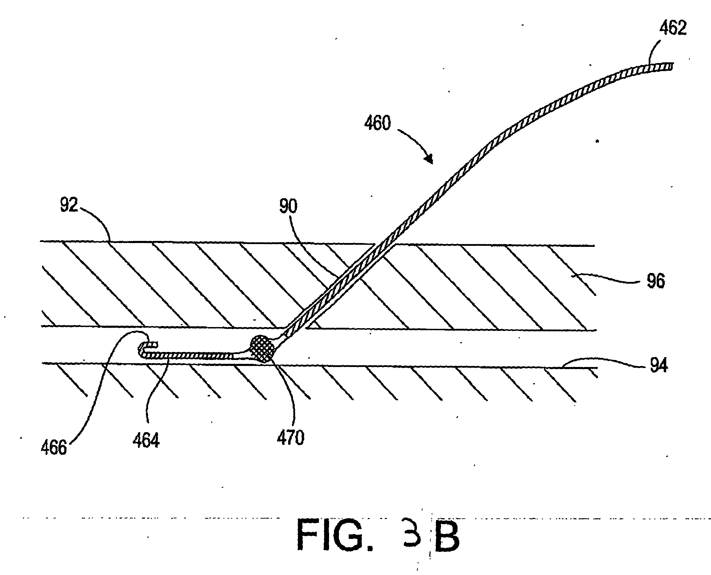

Once the tamp 470 (e.g., covered by the retaining sheath 460) is within the vessel 94, the needle 416 may be removed, and the tamp 470 may be expanded within the vessel 94, as shown in FIG. 3B. For example, the retaining sheath 460 may be retracted at least partially, and/or entirely out of the puncture 90 to expose the tamp 470, whereupon the tamp 470 may automatically expand within the vessel 94. The hub 458 on the retaining sheath 450 may facilitate manipulation of the retaining sheath 450, e.g., during retraction to expose the tamp 470. Although not shown, the tamp 470 may shorten substantially as it expands to minimize occlusion of the vessel 94 when the tamp 470 is in the enlarged condition.

Alternatively, the tamp may be selectively expandable, e.g., using an internal pull wire or other actuator (not shown). Thus, once the tamp is exposed within the vessel 94, the tamp may be expanded, e.g., by pulling the pull wire until the tamp attains a desired enlarged size and/or configuration. In a further alternative, if the tamp is selectively expandable, it may be possible to eliminate the retaining sheath 450, e.g., to reduce the overall profile of the guidewire 460 during insertion into the puncture 90.

Turning to FIG. 3C, after the tamp 470 is expanded, the guidewire 460 may be partially retracted from the vessel 94 until the proximal portion 470a of the tamp 470 contacts the vessel wall and/or substantially seals the puncture 90 from the vessel 94. The delivery sheath 412 may be advanced over the guidewire 460, e.g., before or after the tamp 470 is expanded and/or retracted to seal the puncture 90. As shown, the delivery sheath 412 may be advanced over the guidewire 460 until the distal end 424 contacts the tamp 470, e.g., after the retaining sheath 450 is completely removed. For example, the proximal end 462 of the guidewire 460 may be backloaded through the primary lumen 426 of the delivery sheath 412, and the delivery sheath 412 may be advanced into the puncture 90, the guidewire 460 sliding through the primary lumen 426.

The outlets 431 of the delivery sheath 412 may be disposed a predetermined distance from the distal end 424 of the delivery sheath 412, e.g., at least about five millimeters (5 mm) such that the outlets 431 are disposed within the puncture 90 proximal to the vessel 94 when the distal end 424 contacts the tamp 470. When the distal end 424 contacts the proximal portion 470a of the tamp 470, tactile feedback may provide indication that the outlets 431 are located at the desired position for delivering sealing compound 99.

A source of sealing compound, e.g., dual syringe assembly 40, may be prepared and connected to the side port 432 of the delivery sheath 412 either before or after the delivery sheath 412 is advanced into the puncture 90, similar to the other embodiments described herein. The sealing compound 99 may then be delivered through the secondary lumen 430 and the outlets 431 and into the puncture 90. The sealing compound 99 may flow radially outwardly to permeate at least partially into the tissue surrounding the puncture 90. The delivery sheath 412 may be retracted as the sealing compound 99 is delivered, e.g., to fill the puncture 90 along its length.

Once the puncture 90 is sufficiently filled with the sealing compound 99, the guidewire 460 may be maintained such that the tamp 470 continues to seal the puncture 90 from the vessel 94, e.g., for sufficient time for the sealing compound 99 to at least partially or completely cure. Thereafter (or alternatively immediately after filling the puncture 90), the delivery sheath 412 may be removed entirely from the puncture 90.

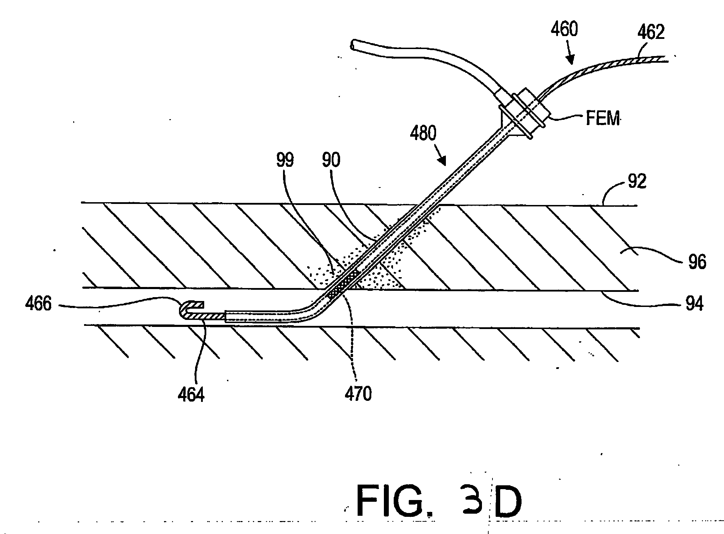

Turning to FIG. 3D, an introducer sheath 480 may then be advanced over the guidewire 460 and into the puncture 90. The introducer sheath 480 may be a conventional introducer sheath similar to those described elsewhere herein, e.g., including a proximal end 482, a distal end 484, and a lumen 486 extending therebetween. The introducer sheath 480 may be advanced over the guidewire 460 until the distal end 484 contacts the tamp 470. Further advancement of the introducer sheath 480 while maintaining or retracting the guidewire 460 may cause the tamp 470 to compress to the contracted condition as it is directed into the lumen 486 of the introducer sheath 480. The guidewire 460 may then be removed entirely from the vessel 94 and puncture 90 by retracting the guidewire 460 through the lumen 486 of the introducer sheath 480.

Alternatively, after filling the puncture 90 with sealing compound 99, the delivery sheath 412 may be removed entirely from the puncture 90, leaving the guidewire 460 in place, e.g., to seal the puncture 90 from the vessel 94 while the sealing compound 99 gels or otherwise cures. Thereafter, the retaining sheath 450 may be advanced back over the tamp 470, e.g., by advancing the retaining sheath 450 over the guidewire 460 while maintaining the guidewire 460 within the vessel 94. This may cause the distal end 454 of the retaining sheath 450 to push against the tamp 470, causing the tamp 470 to compress towards the contracted condition as the tamp 470 enters the lumen 456 of the retaining sheath 450. The retaining sheath 450 and guidewire 460 may then be removed from the vessel 94 and puncture 90, e.g., after an introducer sheath 480 is advanced over the retaining sheath 450 and guidewire 460 into the puncture 90.

Once a distal end 484 of the introducer sheath 480 is disposed within the vessel 94, one or more instruments (not shown) may be advanced through the lumen 486 into the vessel 94, e.g., to perform one or more diagnostic and/or interventional procedures within the patient's body, as is known to those skilled in the art. Upon completing any such procedures, the instrument(s) may be removed from the vessel 94 through the introducer sheath 480.

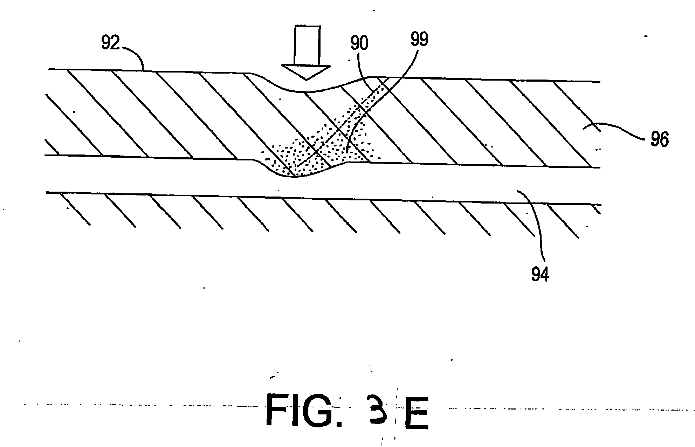

As shown in FIG. 3E, the introducer sheath 480 may be removed from the vessel 94 and puncture 90, allowing the sealing compound 99 to substantially fill the puncture 90, thereby allowing and/or encouraging hemostasis to occur between the vessel 94 and puncture 90. Optionally, external pressure may be applied to the patient's skin 92 during removal of the introducer sheath 480, e.g., to further enhance sealing of the puncture 90 until hemostasis occurs.

## Claims

1. A system (410) for sealing a puncture extending through tissue to a body lumen of a patient, comprising:
a guidewire (460) having proximal and distal ends (462, 464) with an expandable tamp (470) on the distal end (464), the expandable tamp (470) being expandable from a contracted condition to an enlarged condition;
a delivery sheath (412) having a proximal end (422), a distal end (424) sized for insertion through the puncture, a primary lumen (426) extending between the delivery sheath proximal and distal ends (422, 424) and sized for receiving the guidewire (460) therethrough with the expandable tamp (470) in the contracted condition, and a secondary lumen (430) extending from a side port (432) in the delivery sheath proximal end (422) to one or more outlets (431) located between the delivery sheath proximal and distal ends (422, 424); and
a source of sealing compound (40) coupled to the side port (432) of the delivery sheath (412) for delivering sealing compound (99) through the secondary lumen (430) and out the one or more outlets (431) to tissue surrounding the puncture, **characterized in that**
the guidewire (460) and the delivery sheath (412) are arranged such that the delivery sheath (412) may,be advanced over and removed from around the guidewire (460).

2. The system of claim 1, wherein the expandable tamp (470) is biased towards the enlarged condition, the system further comprising a removable constraint (450) for maintaining the expandable tamp (470) in the contracted condition.

3. The system of claim 2, wherein the constraint comprises a tubular member (450) slidable over the guidewire (460).

4. The system of claim 3, wherein the tubular member (450) is slidable within the primary lumen (426) of the delivery sheath (412) such that the tubular member (450) may be retracted through the primary lumen (426) when the expandable tamp (470) is disposed within the body lumen to allow the expandable tamp (470) to expand within the body lumen.

5. The system of claim 1, wherein the expandable tamp (470) comprises an expandable frame.

6. The system of claim 1, wherein the expandable tamp (470) comprises a mesh structure (472).

7. The system of claim 1, wherein the expandable tamp (470) comprises a plurality of struts (472).

8. The system of claim 1, wherein the expandable tamp (470) comprises a substantially nonporous covering.

9. The system of claim 1, wherein the one or more outlets (431) are disposed on a tapered portion (425) of the delivery sheath (412).

10. The system of claim 9, wherein the delivery sheath comprises an extension between its tapered portion and distal end, the extension having a length greater than about five millimeters.

## Patentansprüche

1. Ein System (410) zum Abdichten eines Einstichkanals, der sich durch das Gewebe zu einem Körper-Lumen eines Patienten erstreckt, bestehend aus:
einem Führungsdraht (460), der ein proximales und ein distales Ende (462, 464) aufweist, mit einem ausdehnbaren Tampon (470) am distalen Ende (464), wobei der ausdehnbaren Tampon (470) von einem zusammengezogenen Zustand in einen vergrößerten Zustand ausdehnbar ist;
einer Abgabehülle (412) mit einem proximalen Ende (423) und einem distalen Ende (424), die größenmäßig so beschaffen sind, dass die Abgabehülle (412) durch den Einstichkanal eingeführt werden kann; einem Primärlumen (426), das sich zwischen dem proximalen und distalen Ende (422, 424) der Abgabehülle erstreckt und größenmäßig so beschaffen ist, dass der Führungsdraht (460) mit dem ausdehnbaren Tampon (470) im zusammengezogenen Zustand durchgeführt werden kann sowie einem Sekundärlumen (430), das sich von einer Seitenöffnung (432) am proximalen Endes (422) der Abgabehülle (412) zu einem oder mehreren Öffnungen (431) erstreckt, die zwischen dem proximalen und distalen Ende (422, 424) der Abgabehülle gelegen sind; sowie
einer Quelle für eine Dichtungsmasse (40), die an die Seitenöffnung (432) der Abgabehülle (412) angeschlossen ist, um die Dichtungsmasse (99) durch das Sekundärlumen (430) und aus einem oder mehreren Öffnungen (431) an das den Einstichkanal umgebende Gewebe abzugeben, **dadurch** charakterisiert, dass
der Führungsdraht (460) und die Abgabehülle (412) in einer solchen Weise angeordnet sind, dass die Abgabehülle (412) über dem Führungsdraht (460) vorgeschoben und vom Führungsdraht (460) weg geschoben werden kann.

2. Das System von Anspruch 1, wobei der ausdehnbare Tampon (470) hin zu einem ausgedehnten Zustand tendiert, und wobei das System weiterhin einen entfernbaren Hemmer (450) aufweist, um den ausdehnbaren Tampon (470) im zusammengezogenen Zustand zu halten.

3. Das System von Anspruch 2, wobei der Hemmer ein schlauchförmiges Teil (450) beinhaltet, das über dem Führungsdraht (460) gleiten kann.

4. Das System von Anspruch 3, wobei das schlauchförmige Teil (450) innerhalb des Primärlumens (426) der Abgabehülle (412) so verschiebbar ist, dass das schlauchförmige Teil (450) durch das Primärlumen (426) zurückgezogen werden kann, wenn sich der ausdehnbare Tampon (470) in einem Körper-Lumen befindet, sodass sich der Tampon (470) innerhalb des Körper-Lumens auszudehnen kann.

5. Das System von Anspruch 1, wobei der ausdehnbare Tampon (470) einen ausdehnbaren Rahmen aufweist.

6. Das System von Anspruch 1, wobei der ausdehnbare Tampon (470) eine Maschenstruktur (472) aufweist.

7. Das System von Anspruch 1, wobei der ausdehnbare Tampon (470) eine Vielzahl von Stützstäben (472) aufweist.

8. Das System von Anspruch 1, wobei der ausdehnbare Tampon (470) eine im wesentlichen nicht-poröse Verkleidung enthält.

9. Das System von Anspruch 1, wobei ein oder mehrere Öffnungen (431) auf einem verjüngten Teil (425) der Abgabehülle (412) angeordnet sind.

10. Das System von Anspruch 9, wobei die Abgabehülle eine Verlängerung zwischen ihrem verjüngten Teil und dem distalen Ende besitzt, und wobei die Verlängerung eine Länge aufweist, die mehr als ungefähr fünf Millimeter beträgt.

## Revendications

1. Un système (410), destiné à obturer une perforation s'étendant des tissus cutanés à une lumière corporelle d'un patient, qui comprend :
- un fil-guide (460) doté d'extrémités proximale et distale (462, 464) avec un bouchon obturateur (470) extensible sur l'extrémité distale (464) ; le bouchon obturateur (470) extensible pouvant être déployé dans des positions contractée et détendue,
- une gaine d'acheminement (412) doté d'une extrémité proximale (422), une extrémité distale (424) dimensionnées afin de permettre l'introduction dans la perforation, une lumière primaire (426) s'étendant entre les extrémités proximale (422) et distale (424) de la gaine d'acheminement et dimensionnée afin de recevoir le fil-guide (460) tout le long avec le bouchon obturateur (470) extensible dans un état contracté, et une lumière secondaire (4.30) s'étendant d'un orifice latéral (432) situé dans l'extrémité proximale (422) de la gaine d'acheminement vers un ou plusieurs orifices (431) situés entre les extrémités proximale (422) et distale (424) de la gaine d'acheminement et,
- une source (40) de matériau d'obturation (99) raccordée à un orifice latéral (432) de la gaine d'acheminement (412) destiné à administrer le matériau d'obturation (99) par la lumière secondaire (430) et d'un ou plusieurs orifices (431) vers les tissus entourant la perforation, en quoi
- le fil-guide (460) et la gaine d'acheminement (412) sont disposés de telle sorte que la gaine d'acheminement (412) puisse être avancée et retirée du fil-guide (460).

2. Le système selon la Revendication 1, en quoi le bouchon obturateur (470) extensible est en diagonale en position déployée ; le système comprend également un élément de retenue amovible (450) destiné à maintenir le bouchon obturateur (470) extensible dans un état contracté.

3. Le système selon la Revendication 2, en quoi l'élément de retenue est constitué d'un élément tubulaire (450) pouvant coulisser sur le fil-guide (460).

4. Le système selon la Revendication 3, en quoi l'élément tubulaire (450) peut coulisser à l'intérieur de la lumière primaire (426) de la gaine d'acheminement (412) de telle sorte que l'élément tubulaire (450) puisse être rétracté par la lumière primaire (426) lorsque le bouchon obturateur (470) extensible est placé à l'intérieur de la lumière corporelle afin de permettre au bouchon obturateur (470) extensible de se déployer à l'intérieur de la lumière corporelle.

5. Le système selon la Revendication 1, en quoi le bouchon obturateur (470) extensible est constitué d'un cadre extensible.

6. Le système selon la Revendication 1, en quoi le bouchon obturateur (470) extensible comprend une structure maillée (472).

7. Le système selon la Revendication 1, en quoi le bouchon obturateur (470) extensible est constitué d'une pluralité de montants (472).

8. Le système selon la Revendication 1, en quoi le bouchon obturateur (470) extensible comprend un revêtement essentiellement non poreux.

9. Le système selon la Revendication 1, en quoi un ou plusieurs orifices (431) sont disposés sur une partie effilée (425) de la gaine d'acheminement (412).

10. Le système selon la Revendication 9, en quoi la gaine d'acheminement (412) comprend une extension entre sa partie effilée et l'extrémité distale ; l'extension étant dotée d'une longueur plus importante qu'environ cinq millimètres.
